(19) Europäisches Patentamt
European Patent Office
Office européen des brevets

(11) EP 3 735 230 B1

(12) **EUROPEAN PATENT SPECIFICATION**

(45) Date of publication and mention of the grant of the patent:
**23.04.2025 Bulletin 2025/17**

(21) Application number: **19700427.8**

(22) Date of filing: **02.01.2019**

(51) International Patent Classification (IPC):
***A61K 9/48*** *(2006.01)* ***A61K 31/352*** *(2006.01)*
***A61K 9/28*** *(2006.01)*

(52) Cooperative Patent Classification (CPC):
**A61K 9/4858; A61K 9/2846; A61K 9/2866; A61K 9/288; A61K 9/4866; A61K 9/4875; A61K 31/05; A61K 31/192; A61K 31/352; A61K 45/06**

(86) International application number:
**PCT/GB2019/050008**

(87) International publication number:
**WO 2019/135076 (11.07.2019 Gazette 2019/28)**

(54) **MODIFIED RELEASE COMPOSITION COMPRISING A CANNABINOID**

MODIFIZIERTE FREISETZUNGSZUSAMMENSETZUNG MIT EINEM CANNABINOIDREZEPTOR

COMPOSITION À LIBÉRATION MODIFIÉE CONTENANT UN CANNABINOÏDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.01.2018 GB 201800073**

(43) Date of publication of application:
**11.11.2020 Bulletin 2020/46**

(73) Proprietor: **Jazz Pharmaceuticals Research UK Limited**
**Sittingbourne Kent ME9 8AG (GB)**

(72) Inventors:
- **WILKHU, Jitinder**
  **Sittingbourne**
  **Kent ME9 8AG (GB)**
- **BENDER, Johan**
  **6572 AB Berg en Dal (NL)**
- **COLLINS, Matthew**
  **Sittingbourne**
  **Kent ME9 8AG (GB)**

(74) Representative: **HGF**
**HGF Limited**
**1 City Walk**
**Leeds LS11 9DX (GB)**

(56) References cited:
**WO-A1-2017/072774** **AU-A1- 2016 203 127**
**US-A1- 2016 184 258**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 735 230 B1

## Description

Field of the Invention

The present invention relates to a modified-release oral pharmaceutical composition. The pharmaceutical composition comprises a modified-release agent and a pharmaceutical formulation. The pharmaceutical formulation comprises a cannabinoid.

Background of the Invention

Cannabinoids are lipophilic substances that are known to be poorly soluble in water (less than 1 μg/mL). As an example, CBD is soluble in ethanol (36 mg/mL) and dimethylsulfoxide DMSO (60 mg/mL).

Bioavailability of pharmaceutical substances taken perorally, first of all, depends on the extent to which the pharmaceutically active substance is absorbed from the intestinal environment across the intestinal mucosa. Lipophilic pharmaceutical substances are generally poorly absorbed from the intestinal environment, inter alia because of their poor solubility and/or dispersibility in water. Bioavailability of a pharmaceutical substance taken perorally furthermore depends on the susceptibility of the substance to the so-called first pass effect. Substances absorbed from the intestine, before being distributed throughout the body, have to pass the liver first where they may be metabolised immediately. CBD is generally assumed to be rather susceptible to first-pass liver metabolisation. Oral bioavailability of CBD is low and unpredictable (S. Zhornitsky, S. Potvin, Pharmaceuticals (2012) 5, 529-552). In addition, CBD is an unstable drug (A. J. Poortman, H. Huizer, Forensic Science International (1999) 101, 1-8).

In WO 2012/033478, Self-Emulsifying Drug Delivery Systems (SEDDS) have been used to offer improved administration of cannabinoids.

SEDDS (self-emulsifying drug delivery systems) generally consist of hard or soft capsules filled with a liquid or a gel that consists of lipophilic active pharmaceutical ingredient (API), oil (to dissolve the API) and a surfactant. Upon contact with gastric fluid, the SEDDS spontaneously emulsify due to the presence of surfactants. Many surfactants, however, are lipid based and interact with lipases in the gastro intestinal tract (GIT). This can lead to a reduced capability of the lipid based surfactants to emulsify the API as well as the oil carrier, both reducing bioavailability.

In WO 2015/184127, an alcohol-free formulation comprising a cannabinoid, a polyethylene glycol and propylene glycol is disclosed.

In WO 2012/033478, SEDDS formulations based on Type I, Type II and Type III were utilised.

In PCT/GB2017/051943 (as yet unpublished) a Type IV or Type IV-like formulation comprising a cannabinoid is disclosed.

Other documents relevant to the background of the present invention are CN103110582; CN101040855; US2012/183606; Thumma S Et Al, European Journal of Pharmaceutics and Biopharmaceutics. vol 70, no. 2, 1 October 2008, pp 605-614; and Edward Maa Et Al, Epilepsia, vol. 55, no. 6, 1 June 2014, pp 783-786.

WO 2017/072774 discloses compositions designed for transmucosal administration comprising lipophilic compounds (such as CBD or THC), an amphiphilic polymer (such as poloxamer 407) and a hydrophilic polymer (such as sodium carboxymethylcellulose). AU 2016/203127 and US 2016/184258 disclose oil-based Type I, Type II and Type III SEDDS formulations comprising tetrahydrocannabinol (THC).

The Lipid Formulation Classification System (LFCS) was introduced to help identify the characteristics of lipid systems (C.W. Pouton, Eur. J. Pharm. Sci., 11 (Suppl. 2) (2000), pp. S93-S98). As classified in the LFCS, Type I formulations are oils which require digestion, Type II formulations are water-insoluble self-emulsifying drug delivery systems (SEDDS), Type III systems are SEDDS or self-micro emulsifying drug delivery systems (SMEDDS) or self-nano emulsifying drug delivery systems (SNEDDS) which contain some water-soluble surfactants and/or co-solvents (Type IIIA) or a greater proportion of water soluble components (Type IIIB). Category Type IV represents a recent trend towards formulations which contain predominantly hydrophilic excipient surfactants and co-solvents. Below is a tabular Lipid Formulation Classification System overview taken from US 2015/111939:

| | Content of formulation (wt.-%) | | | | |
|---|---|---|---|---|---|
| Excipients in formulation | Type I | Type II | Type IIIA | Type IIIB | Type IV |
| Oil: triglycerides or mixed mono-and diglycerides | 100 | 40-80 | 40-80 | <20 | - |
| Water-insoluble surfactants (HLB < 12) | - | 20-60 | - | - | 0-20 |
| Water-soluble surfactants (HLB > 12) | - | - | 20-40 | 20-50 | 30-80 |
| Hydrophilic co-solvent | - | - | 0-40 | 20-50 | 0-50 |

A further description of the Lipid Formulation Classification System can also be found in FABAD J. Pharm. Sci., pages 55-64, 2013.

As can be seen in the above table, Type IIIB formulations comprise <20 wt% of oil, based on the total composition. However, it should be noted that, by definition, Type IIIB formulations contain some oil, even if it is only a very small amount.

Oral administration of pharmaceutical products is one of the most preferred routes of administration of pharmaceutical products. However, many pharmaceutically active ingredients (APIs) are degraded in the stomach, for example due to the acidic pH of gastric acid in the stomach and/or due to enzymatic degradation of the exposed API. Additionally, many APIs are irritants of the stomach, hence their delivery should be targeted to avoid release of the API in the stomach. The inventors of the present invention have discovered that cannabinoids, for example cannabidivarin (CBDV), are rapidly metabolised when orally administered, ultimately resulting in poor bioavailability of cannabinoids when administered orally. Therefore another factor that affects bioavailability of cannabinoids taken perorally is the degradation of cannabinoid in the GIT.

Therefore it is desirable to provide a route of oral administration of pharmaceutical products comprising cannabinoids which improves the delivery of cannabinoids.

There also exists a need to provide an oral pharmaceutical composition comprising a cannabinoid that exhibits improved properties such as bioavailability, storage stability and homogeneity.

## Brief Summary of the Invention

The invention is set out in the appended set of claims.

The present invention relates to a novel cannabinoid oral pharmaceutical dosage form, or pharmaceutical composition, comprising a pharmaceutical formulation based on a Type IV or Type IV-like formulation, as classified using the Lipid Formulation Classification System. The oral pharmaceutical composition comprises a pharmaceutical formulation and at least one modified-release agent. By Type IV-like, it is meant that the pharmaceutical formulation comprises no oil, for example no triglycerides or mixed glycerides.

There is provided an oral pharmaceutical composition comprising:

> a core which comprises a pharmaceutical formulation; and
> a shell which comprises at least one modified-release agent selected from the group consisting of a copolymer of methacrylic acid and methacrylate, a copolymer of methacrylic acid and methyl methacrylate, a copolymer of methacrylic acid and ethylacrylate, hydroxypropyl methyl cellulose acetate succinate (HPMC-AS), hydroxypropyl methyl cellulose phthalate (HPMCP), polyvinyl acetate phthalate (PVAP), a copolymer of methyl vinyl ether and maleic anhydride, cellulose acetate phthalate (CAP), cellulose acetate butyrate (CAB), cellulose acetate trimellitate (CAT), cellulose acetate succinate (CAS), ethyl cellulose, methyl cellulose, shellac, gellan gum, zein, alginic acid and waxes;
> wherein the pharmaceutical formulation comprises:
>
> > cannabidiol in an amount of from 10 to 50 wt%;
> > a solvent, in an amount of 20 to 50 wt%, wherein the solvent is selected from the group consisting of diacetin, propylene glycol, triacetin, monoacetin, propylene glycol diacetate, triethyl citrate and mixtures thereof; and
> > at least one poloxamer in an amount of 30 to 60 wt%, wherein the poloxamer is poloxamer 124 or poloxamer 188 or a mixture thereof; and
> > wherein the formulation is oil-free.

This formulation enhances cannabinoid (cannabidiol) bioavailability compared to other formulations based on Type I, Type II, Type IIIA and Type IIIB, as classified by the Lipid Formulation Classification System. Accordingly, the pharmaceutical formulation is not oil-based, i.e. it comprises no oil. Such formulations are classified as Type IV or Type IV-like.

Because the oral pharmaceutical composition according to the invention is a modified-release form, the API is protected from the harsh conditions of the stomach, for example acidity and presence of enzymes. Moreover release of the API to a certain part of the GIT can be targeted, for example the jejunum or colon. Therefore the inventors have found that the composition of the invention enhances bioavailability of orally administered cannabinoids.

By enhancing bioavailability, the total amount of cannabinoid and excipients required during a certain window of time in a treatment of a specific disease may be reduced.

The composition according to the present invention exhibits excellent stability under various, in particular dry, storage conditions.

By enhancing stability, the length of time for which the compositions are fit for consumption, in particular oral administration, may be increased.

Detailed Description of the Invention

**The Cannabinoid**

The formulation according to the present invention comprises cannabidiol (CBD).

The cannabidiol is present in an amount of from 10 to 50 wt%, based on the pharmaceutical formulation, preferably from about 20 to 30 wt%. The cannabidiol may be present in an amount of about 30 wt%.

Preferably, the cannabidiol is synthetically produced or highly purified from its natural source (for example, plant derived recrystallized form, such as a plant derived recrystallized form of CBD). When a highly purified source is used, it is purified such that the cannabidiol is present at greater than 95%, more preferably greater than 98% of the total extract (w/w). Use of a synthetically produced or highly purified cannabidiol is advantageous because these contain relatively low amounts of wax. This assists in prevention of the formation of an oily formulation, increasing physical stability of the formulation and wettability in an aqueous environment.

The unit dose of cannabidiol in the oral pharmaceutical composition may be in the range of from 0.001 to 350 mg, preferably 0.1 to 350 mg, more preferably 1 to 250 mg.

For example, it is envisaged that, when in tablet or capsule unit dose form, the amount of cannabidiol present may be 0.5, 2, 10, 25, 50, 100, 150, 200, 250, 300 or 350 mg.

The amount of cannabidiol present in the formulation may be 20 to 30 wt%, based on the pharmaceutical formulation. It has been found that the formulation is stable and is a solid at room temperature and pressure (defined herein as 20 °C and 1 atm) even when the content of cannabinoid is relatively high, such as 25, 30 or 35 wt%. Without wishing to be bound by theory, it is believed that at least one poloxamer is essential to the stability of the formulation, particularly for high cannabinoid content.

**The Solvent**

The formulation according to the present invention comprises a solvent, wherein the solvent is selected from the group consisting of diacetin, propylene glycol, triacetin, monoacetin, propylene glycol diacetate, triethyl citrate and mixtures thereof.

Diacetin is also known as glycerol diacetate.

Triacetin is also known as 1,2,3-triacetoxypropane, 1,2,3-triacetylglycerol or glycerol triacetate.

Monoacetin is also known as glycerol monoacetate or glycerol acetate.

Triethyl citrate is also known as citric acid ethyl ester.

Propylene glycol, propylene glycol diacetate and triethyl citrate are preferred solvents. Preferably, the solvent is triethyl citrate or propylene glycol. Triethyl citrate is preferably used.

The solvent is present in an amount of from 20 to 50 wt%, most preferably about 20 to 30 wt%. The solvent may be present in an amount of about 25 wt%.

When the solvent used is propylene glycol, it is preferred that it is present in an amount of from about 20 to 30 wt%, based on the pharmaceutical formulation.

When the solvent is triethyl citrate, it is preferred that it is present in an amount of from about 20 to 30 wt%, based on the pharmaceutical formulation.

When only one poloxamer is present, as will be described below, it is preferred that the solvent is present in an amount of from 45 to 50 wt%, based on the pharmaceutical formulation.

The solvent or mixture of solvents according to the claimed invention may be the only solvent in the formulation. For example, the formulation may be substantially water-free and/or substantially alcohol-free. By "substantially water-free" and "substantially alcohol-free", it is meant that the formulation comprises less than 2 wt%, preferably less than 1 wt% water and/or alcohol based on the pharmaceutical formulation.

The formulation is preferably substantially free from ethanol. More preferably the formulation is substantially alcohol-free.

In some embodiments the pharmaceutical composition is used in a paediatric patient, i.e. a patient under 18 years of age. In paediatric patients, it may be preferred that the formulation is substantially alcohol-free.

The formulation is oil-free. The formulation may be free from or comprise no triglycerides, diglycerides or monoglycerides or mixtures thereof derived from glycerol and at least one fatty acid selected from the group consisting of caprylic acid, capric acid, lauric acid, myristic acid, palmitic acid, stearic acid, arachidic acid, behenic acid, lignoceric acid, cerotic acid, myristoleic acid, palmitoleic acid, sapienic acid, oleic acid, elaidic acid, vaccenic acid, linoleic acid, linoelaidic acid, $\alpha$-linolenic acid, arachidonic acid, eicosapentaenoic acid, erucic acid and docosahexaenoic acid and mixtures thereof. Preferably the formulation may be free from or comprise no triglycerides, diglycerides or monoglycerides or mixtures thereof.

The formulation may be free from hydrogenated vegetable oils, nut oils, anise oil, soybean oil, hydrogenated

soybean oil, apricot kernel oil, corn oil, olive oil, peanut oil, almond oil, walnut oil, cashew oil, rice bran oil, poppy seed oil, cottonseed oil, canola oil, sesame oil, hydrogenated sesame oil, coconut oil, flaxseed oil, cinnamon oil, clove oil, nutmeg oil, coriander oil, lemon oil, orange oil, safflower oil, cocoa butter, palm oil, palm kernel oil, sunflower oil, rapeseed oil, castor oil, hydrogenated castor oil, polyoxyethylene castor oil derivatives, borage oil, beeswax, lanolin, petroleum jelly, mineral oil and light mineral oil.

More preferably the formulation may be free from triglycerides, diglycerides or monoglycerides or mixtures thereof derived from glycerol and caprylic acid, capric acid, lauric acid, myristic acid, palmitic acid, stearic acid, arachidic acid, behenic acid, lignoceric acid, cerotic acid, myristoleic acid, palmitoleic acid, sapienic acid, oleic acid, elaidic acid, vaccenic acid, linoleic acid, linoelaidic acid, α-linolenic acid, arachidonic acid, eicosapentaenoic acid, erucic acid and docosahexaenoic acid and mixtures thereof, hydrogenated vegetable oils, nut oils, anise oil, soybean oil, hydrogenated soybean oil, apricot kernel oil, corn oil, olive oil, peanut oil, almond oil, walnut oil, cashew oil, rice bran oil, poppy seed oil, cottonseed oil, canola oil, sesame oil, hydrogenated sesame oil, coconut oil, flaxseed oil, cinnamon oil, clove oil, nutmeg oil, coriander oil, lemon oil, orange oil, safflower oil, cocoa butter, palm oil, palm kernel oil, sunflower oil, rapeseed oil, castor oil, hydrogenated castor oil, polyoxyethylene castor oil derivatives, borage oil, beeswax, lanolin, petroleum jelly, mineral oil and light mineral oil.

**The Poloxamer**

The formulation according to the present invention comprises at least one poloxamer, wherein the poloxamer is poloxamer 124 or poloxamer 188 or a mixture thereof.

A poloxamer is defined according to formula (II)

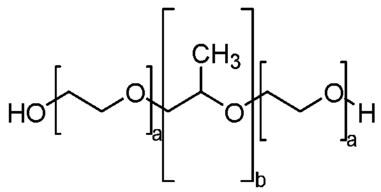

(II)

wherein a is an integer of from 10 to 110 and b is an integer of from 20 to 60.

When a is 12 and b is 20, this is known as poloxamer 124.

When a is 80 and b is 27, this is known as poloxamer 188.

The total amount of poloxamer present is in an amount of from 30 to 60 wt%, based on the pharmaceutical formulation. More preferably the total amount of poloxamer present is from about 40 to about 50 wt%. The total amount of poloxamer present may be about 45 wt%.

When the formulation comprises poloxamer 124 and poloxamer 188, the amount of poloxamer 124 may be 5 wt% and the amount of poloxamer 188 may be 40 wt%, based on the pharmaceutical formulation.

In some cases, the formulation may comprise only one poloxamer, wherein the poloxamer is poloxamer 188.

It has been found that the formulation of the invention has excellent rehydration properties. The formulation rehydrates rapidly and homogeneously. Upon rehydration the formulation has excellent release properties.

It has been found that the formulation of the invention has excellent stability. Without wishing to be bound by theory, it is believed that the presence of at least one poloxamer in the formulation affords excellent stability.

**Modified-release and Oral Dosage Forms**

The oral pharmaceutical dosage form or pharmaceutical composition according to the invention comprises a modified-release agent selected from the group consisting of a copolymer of methacrylic acid and methacrylate, a copolymer of methacrylic acid and methyl methacrylate, a copolymer of methacrylic acid and ethylacrylate, hydroxypropyl methyl cellulose acetate succinate (HPMC-AS), hydroxypropyl methyl cellulose phthalate (HPMCP), polyvinyl acetate phthalate (PVAP), a copolymer of methyl vinyl ether and maleic anhydride, cellulose acetate phthalate (CAP), cellulose acetate butyrate (CAB), cellulose acetate trimellitate (CAT), cellulose acetate succinate (CAS), ethyl cellulose, methyl cellulose, shellac, gellan gum, zein, alginic acid and waxes.

The modified-release agent may be an acid-resistant agent.

The modified-release agent may be an enteric agent.

The oral pharmaceutical composition may be in an oral dosage form selected from the group consisting of mucoadhesive gel, a tablet, a powder, a liquid gel capsule, a solid capsule, an oral solution, granule or extrudates.

The oral pharmaceutical composition comprises a core and a shell, wherein the core comprises the pharmaceutical formulation and the core is substantially, for example completely, surrounded by the shell, and wherein the shell comprises the modified-release agent. The shell may be a capsule. The shell may be a coating. The oral pharmaceutical composition may comprise at least one intermediate layer positioned between the core and the shell. Examples of the intermediate layer include a capsule.

A preferred group of oral dosage forms is the group consisting of a gel capsule and a solid capsule. When the oral pharmaceutical composition is in the dosage form of a capsule, the pharmaceutical formulation is contained in the capsule and the capsule comprises the modified-release agent (either as part of the capsule material, or the capsule comprises a coating which comprises the modified-release agent).

The capsule may comprise the modified-release agent as part of the capsule material, for example a capsule which is made from a material that comprises a modified-release agent.

The capsule may be coated with a coating comprising the modified-release agent, for example a capsule which is not made from a material that comprises a modified-release agent, but which is coated with a coating that comprises the modified-release agent.

The oral dosage form may be a capsule which comprises a modified-release agent, for example a capsule which is made from a material that comprises a modified-release agent, and which is coated with a coating that comprises the modified-release agent.

The oral dosage form may be an acid-resistant dosage form.

The oral dosage form may be an enteric dosage form, such as an enteric capsule.

The pharmaceutical formulation according to the present invention may be filled into capsules with a modified-release coating, wherein the coating comprises at least one a modified-release agent.

The pharmaceutical formulation according to the present invention may be filled into modified-release capsules which comprise the least one modified-release agent as part of the capsule material.

Preferably the modified-release capsule comprises hydroxypropyl methyl cellulose acetate succinate (HPMC-AS).

Preferably the modified-release capsule comprises a coating comprising cellulose acetate phthalate (CAP).

## Additional Agents

The formulation may additionally comprise a flavouring agent, such as peppermint.

The formulation may additionally comprise a sweetener, such as sucrose.

The formulation may further comprise an antioxidant, preferably in an amount of from about 0.001 to 5 wt%, more preferably about 0.001 to 2.5 wt%, based on the pharmaceutical formulation.

The antioxidant may be selected from the group consisting of butylated hydroxytoluene, butylated hydroxyl anisole, alpha- tocopherol (Vitamin E), ascorbyl palmitate, ascorbic acid, sodium ascorbate, ethylenediamino tetraacetic acid, cysteine hydrochloride, citric acid, sodium citrate, sodium bisulfate, sodium metabisulfite, lecithin, propyl gallate, sodium sulfate, monothioglycerol and mixtures thereof.

A preferred group of antioxidants is alpha- tocopherol (Vitamin E), monothioglycerol, ascorbic acid, citric acid and mixtures thereof. A preferred antioxidant is alpha- tocopherol (Vitamin E).

## Preferred Formulations

It is preferred that the type IV oral formulation according to the invention is a solid at room temperature and pressure, i.e. preferably the formulation is a solid at 20 °C and 1 atm. Such formulations are typically fluid during manufacture, solid at room temperature and become fluid again at 37 °C. For the purposes of the invention, a gel is considered to be a solid.

The formulation may comprise about 20 to 30 wt% solvent and two poloxamers, wherein the total amount of poloxamer is about 30 to 60 wt%, based on the pharmaceutical formulation.

Preferably the formulation comprises about 20 to 30 wt% cannabinoid, about 20 to 30 wt% solvent and two poloxamers wherein the poloxamers are poloxamer 124 and poloxamer 188, wherein the total amount of poloxamer is about 30 to 60 wt%, based on the pharmaceutical formulation.

Preferably the formulation comprises CBD; at least two poloxamers, wherein the poloxamers are poloxamer 124 and poloxamer 188; and a solvent, wherein the solvent is triethyl citrate. More preferably the formulation comprises about 20 to 30 wt% CBD; about 20 to 30 wt% triethyl citrate; and two poloxamers, wherein the poloxamers are poloxamer 124 and poloxamer 188, wherein the total amount of poloxamer is about 30 to 60 wt%, based on the pharmaceutical formulation.

In a highly preferred formulation, the formulation comprises about 20 to 30 wt% CBD; about 20 to 30 wt% triethyl citrate; an anti-oxidant, wherein the antioxidant is alpha-tocopherol; and two poloxamers, wherein the poloxamers are poloxamer 124 and poloxamer 188, wherein the total amount of poloxamer is about 40 to 50 wt%, based on the pharmaceutical formulation. In this preferred formulation, the formulation is in the form of an oral dosage form, wherein

the oral dosage form is a capsule; and the capsule comprises the modified-release agent.

The following represent preferred formulations according to the invention that are capable of forming a gel at body temperature.

A preferred oral pharmaceutical formulation (solid gel at room temperature) comprises

25 wt% cannabidiol;
34 wt% poloxamer 124;
15 wt% poloxamer 188; and
26 wt% propylene glycol.

A further preferred oral pharmaceutical formulation (Gel at room temperature) comprises

25 wt% cannabidiol;
34 wt% poloxamer 124;
15 wt% poloxamer 188; and
26 wt% diacetin.

A further preferred oral pharmaceutical formulation (Solid at room temperature) comprises

25 wt% cannabidiol;
35 wt% poloxamer 124;
20 wt% poloxamer 188; and
20 wt% propylene glycol.

A further preferred oral pharmaceutical formulation (Gel at room temperature) comprises

35 wt% cannabidiol;
28 wt% poloxamer 124;
16 wt% poloxamer 188; and
22 wt% propylene glycol.

A further preferred oral pharmaceutical formulation (Solid at room temperature) comprises

12.5 wt% cannabidiol;
38 wt% poloxamer 124;
19 wt% poloxamer 188; and
30 wt% propylene glycol.

A further preferred oral pharmaceutical formulation (Solid at room temperature) comprises

25 wt% cannabidiol;
35 wt% poloxamer 124;
20 wt% poloxamer 188; and
20 wt% triethyl citrate.

**Treatment**

The composition is for use in therapy, preferably for use in paediatric epilepsy.

The composition may also be used in the treatment of a disease or disorder selected from the group consisting of Dravet Syndrome, Lennox Gastaut Syndrome, myocolonic seizures, juvenile myocolonic epilepsy, refractory epilepsy, schizophrenia, juvenile spasms, West syndrome, infantile spasms, refractory infantile spasms, tuberous sclerosis complex, brain tumors, neuropathic pain, cannabis use disorder, post-traumatic stress disorder, anxiety, early psychosis, Alzheimer’s Disease, and autism.

As already stated, cannabidiol is preferred for use in the present invention. Cannabidiol can be used in the treatment of atonic, absence or partial seizures, in particular, simple or complex seizures. It is particularly effective in reducing seizures in patients suffering with etiologies that include: Lennox-Gastaut Syndrome; Tuberous Sclerosis Complex; Dravet Syndrome; Doose Syndrome; CDKL5; Dup15q; , Jeavons syndrome; Myoclonic Absence Epilepsy; Neuronal ceroid lipofuscinoses (NCL) and brain abnormalities.

In addition, a composition comprising CBDV and/or CBDA can be used in the treatment of autism spectrum disorders, in particular Rett syndrome, Fragile X syndrome, Angelman syndrome, ADHD and hyperkinetic disorders, such as Tourette syndrome and dystonias. Thus, the composition comprising CBDV and/or CBDA can be useful in a method of treatment of such disorders.

The composition of the invention may be useful in a method of treating a patient having a disorder selected from the group consisting of Dravet Syndrome, Lennox Gastaut Syndrome, myoclonic seizures, juvenile myoclonic epilepsy, refractory epilepsy, schizophrenia, juvenile spasms, West syndrome, infantile spasms, refractory infantile spasms, tuberous sclerosis complex, brain tumors, neuropathic pain, cannabis use disorder, post-traumatic stress disorder, anxiety, early psychosis, Alzheimer's Disease, and autism.

When cannabidiol is used in the composition, the composition may be useful in a method of treatment of atonic, absence or partial seizures in a patient, in particular, simple or complex seizures. It is particularly effective in a method of reducing seizures in patients suffering with etiologies that include: Lennox-Gastaut Syndrome; Tuberous Sclerosis Complex; Dravet Syndrome; Doose Syndrome; CDKL5; Dup15q; , Jeavons syndrome; Myoclonic Absence Epilepsy; Neuronal ceroid lipofuscinoses (NCL) and brain abnormalities.

The method of treatments comprise administering a patient with a therapeutically effective amount of a composition or of a cannabinoid in a composition according to the present invention.

**Definitions**

"Cannabinoids" are a group of compounds including the endocannabinoids, the phytocannabinoids and those which are neither endocannabinoids nor phytocannabinoids, hereinafter "syntho-cannabinoids".

"Endocannabinoids" are endogenous cannabinoids, which are high affinity ligands of CB1 and CB2 receptors.

"Phytocannabinoids" are cannabinoids that originate in nature and can be found in the cannabis plant. The phytocannabinoids can be present in an extract including a botanical drug substance, isolated, or reproduced synthetically.

"Syntho-cannabinoids" are those compounds capable of interacting with the cannabinoid receptors (CB1 and/or CB2) but are not found endogenously or in the cannabis plant. Examples include WIN 55212 and rimonabant.

An "isolated phytocannabinoid" is one which has been extracted from the cannabis plant and purified to such an extent that all the additional components such as secondary and minor cannabinoids and the non-cannabinoid fraction have been removed.

A "synthetic cannabinoid" is one which has been produced by chemical synthesis. This term includes modifying an isolated phytocannabinoid, by, for example, forming a pharmaceutically acceptable salt thereof.

A "substantially pure" cannabinoid is defined as a cannabinoid which is present at greater than 95% (w/w) pure. More preferably greater than 96% (w/w) through 97% (w/w) thorough 98% (w/w) to 99% % (w/w) and greater.

A "highly purified" cannabinoid is defined as a cannabinoid that has been extracted from the cannabis plant and purified to the extent that other cannabinoids and non-cannabinoid components that are co-extracted with the cannabinoids have been substantially removed, such that the highly purified cannabinoid is greater than or equal to 95% (w/w) pure.

A "botanical drug substance" or "BDS" is defined in the Guidance for Industry Botanical Drug Products Draft Guidance, August 2000, US Department of Health and Human Services, Food and Drug Administration Centre for Drug Evaluation and Research as: "A drug derived from one or more plants, algae, or microscopic fungi. It is prepared from botanical raw materials by one or more of the following processes: pulverisation, decoction, expression, aqueous extraction, ethanolic extraction or other similar processes."

A botanical drug substance does not include a highly purified or chemically modified substance derived from natural sources. Thus, in the case of cannabis, BDS derived from cannabis plants do not include highly purified Pharmacopoeial grade cannabinoids.

An "oil" is typically defined as a single compound or a mixture of compounds that are both hydrophobic and lipophilic. Exemplary oils include triglycerides, diglycerides, monoglycerides, fatty acids and fatty acid esters. Triglycerides, diglycerides and monoglycerides are esters derived from glycerol and three, two or one fatty acids. Diglycerides and triglycerides may have the same or they may have different fatty acids for each ester bond. Exemplary fatty acids include carboxylic acids with a saturated or unsaturated, linear or branched carbon chains, such as caprylic acid, capric acid, lauric acid, myristic acid, palmitic acid, stearic acid, arachidic acid, behenic acid, lignoceric acid, cerotic acid, myristoleic acid, palmitoleic acid, sapienic acid, oleic acid, elaidic acid, vaccenic acid, linoleic acid, linoelaidic acid, α-linolenic acid, arachidonic acid, eicosapentaenoic acid, erucic acid and docosahexaenoic acid. Exemplary mixtures of oils include plant and animal fats and waxes such as vegetable oils, hydrogenated vegetable oils, nut oils, anise oil, soybean oil, hydrogenated soybean oil, apricot kernel oil, corn oil, olive oil, peanut oil, almond oil, walnut oil, cashew oil, rice bran oil, poppy seed oil, cottonseed oil, canola oil, sesame oil, hydrogenated sesame oil, coconut oil, flaxseed oil, cinnamon oil, clove oil, nutmeg oil, coriander oil, lemon oil, orange oil, safflower oil, cocoa butter, palm oil, palm kernel oil, sunflower oil,

rapeseed oil, castor oil, hydrogenated castor oil, polyoxyethylene castor oil derivatives, borage oil, beeswax, lanolin, petroleum jelly, mineral oil and light mineral oil. For the purposes of the present invention cannabinoids are not considered to be oils.

An "alcohol" has its standard meaning within the art. It includes ethanol, propanol etc.

"Room temperature and pressure" is defined herein as 20 °C and 1 atm.

"Modified-release" as used herein refers to the process and result of modifying an oral dosage form or pharmaceutical composition to release a drug with a delay after its oral administration, or for a prolonged period of time, or to a specific target. For the purposes of the present invention, hydroxypropyl methyl cellulose (HPMC) is not considered a modified-release agent.

"Acid-resistant" or "acid resistance" as used herein means that the oral dosage form or pharmaceutical composition does not dissolve (or disintegrate) substantively in solutions with a pH of less than 5, preferably less than 4, more preferably less than 3, even more preferably less than 2; but does dissolve in solutions with a pH of more than 5. For example, the oral dosage form or pharmaceutical composition may not dissolve in gastric acid.

The term "enteric" means that the oral dosage form or pharmaceutical composition does not dissolve (or disintegrate) substantively in gastric acid (either in the fed or fasted state) or in the stomach but does dissolve in the intestines (small intestine, large intestine). For example, the oral dosage form or pharmaceutical composition may dissolve substantively in the jejunum or colon, etc.

Examples

### 1. Analytical procedures, cannabinoids and excipients used in the examples

#### 1.1. Rehydration (RH) procedure

A type IV oral pharmaceutical formulation (OPF) comprising at least one cannabinoid, at least one solvent and at least one poloxamer was rehydrated by adding 20 mL water for injections at room temperature (RH-RT) or by adding 20 mL water for injections at 37 °C (RH-37) in Class-3 glass colourless transparent vials. The vials were vortexed for 10 seconds.

#### 1.2. Test for appearances of OPF

The viscosity, homogeneity and clarity of the OPF was checked visually.

#### 1.3. Appearance of rehydrated OPF

After rehydration, the formulation is checked visually on homogeneity and presence of particles and/or non-rehydrated OPF. The presence of foam is an indication that enough poloxamer is used to rehydrate the cannabinoid(s).

#### 1.4. Release of cannabinoid in rehydration fluid

The release of cannabinoid in the rehydration fluid was tested as follows:
Rehydrated OPF was submitted for HPLC analysis. Equipment: HPLC system with variable wavelength UV detector or diode array detector. Column: Ace C18-AR 150 x 4.6 mm , 3 μm. Pre-Column: Ace C18-AR Guard Cartridge. Mobile Phase: Acetonitrile: 0.25% acetic acid (62%: 38%). Column Temperature: 38°C. Flow Rate: 1.0 ml min-1. Detection: 220 nm. Injection Volume: 10 μl. Run Time 25 minutes. Sample preparation: accurately prepare test samples at an approximate concentration of 0.15 mg/ml in triplicate. Samples may be prepared at a higher concentration to ensure accurate quantification of related substances or degradants. 0.1 mL rehydrated OPF was diluted with 10 mL ethanol; 10 μL was injected into the HPLC system.

#### 1.5. Cannabinoids

CBD: synthetic, plant derived CBD containing waxes and plant derived recrystallized CBD (CBD-r). Plant derived CBDV and synthetic CBDV.

#### 1.6. Excipients

Lutrol L44 (BASF, poloxamer 124: P124), Lutrol F68 (BASF, poloxamer 188: P188), Lutrol F87 (BASF, poloxamer 237: P237), Lutrol F108 (BASF, poloxamer 338: P338), Lutrol F127 (BASF, poloxamer 407, P407), glycerol (Sigma: gly), diacetin (Sigma: di), triacetin (Sigma: tri), propylene glycol (Sigma: PG), ethanol (Fischer), propylene glycol diacetate

(Sigma: PGDA), triethyl citrate (Sigma: TEC).

### 1.7. Melt Procedure

Unless otherwise stated all formulations were produced using the following method. The excipients and cannabinoids are weighed into a vessel and are heated until molten. Upon cooling the gel is filled into capsules or vials by weight. The viscosity of the gel is a function of temperature which enables the flexibility of filling into HPMC, Gelatin and soft-Gelatin capsules.

Alternatively, gel based formulations can be manufactured where the excipients and cannabinoids can be dissolved into an organic solvent such as, ethanol, methanol, propanol and filled into glass vials with a process step of evaporating the organic solvent off to leave the gel in the vial.

### 2. Stability

Stability of OPF was executed according to ICH Guidance Q1A - Q1F. Samples were stored at 25°C ± 2°C/60% RH ± 5%, 30°C ± 2°C/65% RH ± 5% RH and 40°C ± 2°C/75% RH ± 5%. Stability of OPF was assessed by chemical analysis and appearance described above. Chemical analysis was performed by a stability indicating HPLC method, described above. The number of repeat experiments for each time point was 3, except at 6 months, when 6 repeat experiments were conducted. Sample preparation: 0.1 mL rehydrated OPF was diluted with 10 mL ethanol; 10 µL was injected into the HPLC system.

The following formulation was prepared for use in the stability study.

Type IV formulation (150 mg/capsule): 30% w/w CBD; 5% w/w P124; 40% w/w P188; and 25% w/w triethyl citrate.

The purpose of stability testing is to provide evidence on how the quality of a drug product varies with time under the influence of a variety of environmental factors such as temperature and humidity. In order to illustrate that the Type IV formulations according to the invention exhibit excellent stability, stability of OPF was executed according to ICH Guidance Q1A - Q1F.

The results of the stability study are represented in Tables 1-3 below. Table 1 presents the data for samples stored at 25°C ± 2°C/60% RH ± 5%. Table 2 presents the data for samples stored at 30°C ± 2°C/65% RH ± 5% RH. Table 3 presents the data for samples stored at 40°C ± 2°C/75% RH ± 5%.

Table 1

| | **Time Point (Months)** | | | |
|---|---|---|---|---|
| | **0** | **3** | **6** | **7** |
| **CBD Content (mg/Capsule)** | 149.13 | 149.56 | 149.54 | 147.70 |
| **(% of Initial CBD Content)** | 100.00 | 100.3 | 100.3 | 99.0 |

Table 2

| | **Time Point (Months)** | | | |
|---|---|---|---|---|
| | 0 | 3 | 6 | 7 |
| | **Time Point (Months)** | | | |
| | 0 | 3 | 6 | 7 |
| **CBD Content (mg/Capsule)** | 149.13 | 150.12 | 148.58 | 147.05 |
| **(% of Initial CBD Content)** | 100.00 | 100.7 | 99.6 | 98.6 |

Table 3

| | **Time Point (Months)** | | |
|---|---|---|---|
| | 0 | 3 | 6 |
| **CBD Content (mg/Capsule)** | 149.13 | 148.02 | 146.20 |
| **(% of Initial CBD Content)** | 100.00 | 99.3 | 98.0 |

As shown in Tables 1-3, the Type IV formulations according to the invention exhibit excellent stability, even under strenuous conditions, such as 40°C ± 2°C/75% RH ± 5%. Even under storage conditions of 40°C ± 2°C/75% RH ± 5%, 98% of the initial CBD content was recovered after 6 months.

In summary, it has been shown that a Type IV formulation according to the invention, exhibits excellent stability.

### 3. Production of Modified-Release Pharmaceutical Compositions

The following pharmaceutical formulation was prepared for use in the pharmaceutical composition in this study.

30% w/w CBD; 5% w/w P124; 40% w/w P188; and 25% w/w triethyl citrate.

The following modified-release coating was prepared for use in the pharmaceutical composition in this study.

15% cellulose acetate phthalate (CAP), 3% propylene glycol, 1% tween 80, 40.5% ethanol, 40.5% acetone.

The excipients and cannabinoids in the pharmaceutical formulation are weighed into a vessel and are heated until molten. Upon cooling the gel is filled into capsules or vials by weight. The viscosity of the gel is a function of temperature which enables the flexibility of filling into HPMC, Gelatin and soft-Gelatin capsules. After the gel is filled into capsules the capsules are sealed with 50/50 water/ethanol solution.

Alternatively, gel based formulations can be manufactured where the excipients and cannabinoids can be dissolved into an organic solvent such as, ethanol, methanol, propanol and filled into glass vials with a process step of evaporating the organic solvent off to leave the gel in the vial.

After the gel based pharmaceutical formulation or capsule containing the gel has been formed, it is triple coated with the above modified-release coating and dried at 40°C between each coat.

### 4. Dissolution Study

Dissolution of pharmaceutical compositions comprising a modified-release agent was measured according to USP 2 Apparatus with a paddle set at 75 rpm. In the first example the modified-release coated capsules described above were placed in a 0.1M HCl solution (pH 1.1). In the second example the modified-release coated capsules described above were placed in a phosphate buffer solution containing 3% labrasol (pH 6.8). The number of repeat experiments was 6. The concentration of CBD in aliquots taken from the above described solutions was measured at set time intervals. This is indicative of dissolution of the capsule at pH 1.1 and 6.8. The CBD was quantified using HPLC method as described previously. The results of the study are presented below in Table 4.

Table 4

| | **CBD content at time point (% of initial concentration)** | |
|---|---|---|
| **Time Point (mins)** | **0.1M HCl (pH 1.1)** | **Phosphate buffer solution with 3% labrasol (pH 6.8)** |
| Pure solution (0) | 0 | 0 |
| 45 | 1 | 100 |
| 60 | 1 | 101 |
| 120 | 2 | 102 |

As shown in Table 4, the modified-release pharmaceutical composition according to the invention did not substantially dissolve in the acidic solution (pH 1.1) and did not substantially release of the cannabinoid after 2 hours. In contrast the modified-release pharmaceutical composition according to the invention rapidly released 100% of the cannabinoid after 45 minutes in the solution of pH 6.8. These data suggest that the oral pharmaceutical composition according to the invention will exhibit excellent bioavailability and delivery of the cannabinoid can be targeted.

### 5. Bioavailability

In order to illustrate that the Type IV formulations according to the invention exhibit improved bioavailability relative to Type I and Type III formulations, a comparison was made and bioavailability for each formulation measured. The results of the bioavailability study are represented in Table 5 below.

The outcome of the study is also depicted in Figure 1. As can be seen, the Type IV formulation, according to the present invention exhibits improved bioavailability compared to Type I and Type III formulations having the same concentration of CBD. As shown in Table 5, the result of subject 50 appears to be an anomaly because it falls outside of the general trend of improved bioavailability. This is clearly shown in Figure 1, despite inclusion of the anomaly.

In summary, it has been shown that a Type IV formulation, as classified by the Lipid Formulation Classification

System, exhibits improved bioavailability for CBD.

### 5.1. Details of the PK study for measurement of bioavailability

Beagle dogs (supplied by Charles River UK) received oral capsule doses at a target level of 15 mg/kg. Capsules used were size '0' gelatine capsules and the animals received a 100 mL water flush after each capsule was administered. The volume of blood taken at each sampling time was 2 mL and were collected mostly from the jugular vein. On a few occasions, cephalic vein samples were collected. The sampling times were: 0.5, 1, 1.5, 2, 2.5, 3, 4, 5, 6, 8, 12 and 24 h post-dose. The determination of CBD, 6-OH CBD, THC and 11 OH THC in dog plasma was performed by protein precipitation with reverse phase liquid chromatography with tandem mass spectrometric detection. The LLOQ of CBD was 1 ng/ml and all metabolites had an LLOQ of 0.5 ng/ml.

The human equivalent dose (HED) can be estimated using the following formula:

$$\text{HED} = \text{Animal dose (mg/kg)}\ \textit{multiplied by}\ \frac{\text{Animal } K_m}{\text{Human } K_m}$$

The $K_m$ for a dog is 20 and the $K_m$ for a human is 37.

Thus, for a human a 15 mg/kg dose in a dog equates to a human dose of about 8.1 mg/kg.

### 5.2. Formulations for measurement of bioavailability

Diacetin was weighed by weight into a vial followed by P124 directly on top. The P188 was weighed and added to the vessel containing the diacetin and P124. Finally, the desired amount of CBD is weighed and added to the vessel and heated (100 °C) until molten with a vortex to ensure a homogenous gel. Upon cooling (30-40 °C) the gel is filled into capsules or vials by weight. The viscosity of the gel is a function of temperature which enables the flexibility of filling into HPMC, Gelatin and soft-Gelatin capsules. At room temperature, low CBD dose gels were solid whereas the higher loaded CBD formulations remained a gel.

The following formulations were prepared for use in the PK study.

Type IV Gel (125 mg/g): 12.5% w/w CBD; 38% w/w P124; 19% w/w P188; and 30% w/w diacetin. Release = 99.3%. Appearance = solid gel.

Type IV Gel (250 mg/g): 25% w/w CBD; 34% w/w P124; 15% w/w P188; and 26% w/w diacetin. Release = 97.4%. Appearance = clear gel.

In both gel formulations, the CBD used was a highly purified form.

Type III(i) SEDDS (250 mg/g): CBD formulated with 15 wt% oil, 45 wt% water soluble surfactants and 40 wt% hydrophilic cosolvent.

Type III(ii) SEDDS (250 mg/g): CBD formulated with 31 wt% oil, 45 wt% water soluble surfactants and 24 wt% hydrophilic cosolvent.

Table 5

| Estimated bioavailabilities based on AUC(0-t) data for CBD | | | | | | | | | | | | | | | |
|---|---|---|---|---|---|---|---|---|---|---|---|---|---|---|---|
| | | Subject | | | | | | | | | | | | | |
| | | 47 | 48 | 49 | 50 | 57 | 58 | 59 | 60 | 61 | 62 | 63 | N | Mean | SD |
| Analyte | Oral Formulation | Bioavailability_using_AUCt_for_CBD | | | | | | | | | | | | | |
| Type I | Control Oil based (125 mg/g) | 4.43 | 2.95 | | | | 2.11 | 1.67 | 2.43 | | | | 5 | 2.72 | 1.07 |
| Type III(i) | SEDDS (250 mg/g) | 19.9 | 46.7 | | | | 15.5 | 20.0 | 27.0 | | | | 5 | 25.8 | 12.4 |
| Type III(ii) | SEDDS (250 mg/g) | | | 9.00 | 11.7 | 14.6 | | | | 6.62 | 6.65 | 16.3 | 6 | 10.8 | 4.09 |
| Type IV | Gel (125 mg/g) | | | 20.4 | | 31.1 | | | | 10.3 | 25.9 | 22.3 | 5 | 22.0 | 7.70 |
| Type IV | Gel (250 mg/g) | | | 37.2 | 17.3 | 38.0 | | | | 55.7 | 53.5 | 44.3 | 6 | 41.0 | 13.9 |

## Claims

1. An oral pharmaceutical composition comprising:

    a core which comprises a pharmaceutical formulation; and
    a shell which comprises at least one modified-release agent selected from the group consisting of a copolymer of methacrylic acid and methacrylate, a copolymer of methacrylic acid and methyl methacrylate, a copolymer of methacrylic acid and ethylacrylate, hydroxypropyl methyl cellulose acetate succinate (HPMC-AS), hydroxypropyl methyl cellulose phthalate (HPMCP), polyvinyl acetate phthalate (PVAP), a copolymer of methyl vinyl ether and maleic anhydride, cellulose acetate phthalate (CAP), cellulose acetate butyrate (CAB), cellulose acetate trimellitate (CAT), cellulose acetate succinate (CAS), ethyl cellulose, methyl cellulose, shellac, gellan gum, zein, alginic acid and waxes;
    wherein the pharmaceutical formulation comprises:

        cannabidiol in an amount of from 10 to 50 wt%;
        a solvent, in an amount of 20 to 50 wt%, wherein the solvent is selected from the group consisting of diacetin, propylene glycol, triacetin, monoacetin, propylene glycol diacetate, triethyl citrate and mixtures thereof; and
        at least one poloxamer in an amount of 30 to 60 wt%, wherein the poloxamer is poloxamer 124 or poloxamer 188 or a mixture thereof; and
        wherein the formulation is oil-free.

2. The oral pharmaceutical composition according to claim 1, wherein the pharmaceutical formulation comprises two poloxamers.

3. The oral pharmaceutical composition according to claim 2, wherein the two poloxamers are poloxamer 124 and poloxamer 188.

4. The oral pharmaceutical composition according to any one of the preceding claims, wherein the solvent is selected from the group consisting of propylene glycol, propylene glycol diacetate, triethyl citrate and mixtures thereof.

5. The oral pharmaceutical composition according to any one of the preceding claims, wherein the solvent is selected from the group consisting of propylene glycol, triethyl citrate and mixtures thereof.

6. The oral pharmaceutical composition according to any one of the preceding claims, wherein the solvent is triethyl citrate.

7. The oral pharmaceutical composition according to any one of the preceding claims, wherein the solvent is present in an amount of 20 to 30 wt%.

8. The oral pharmaceutical composition according to any one of the preceding claims, wherein the cannabidiol is synthetic or highly purified from its natural source.

9. The oral pharmaceutical composition according to any one of the preceding claims, wherein the cannabidiol is present in an amount of from 20 to 30 wt%.

10. The oral pharmaceutical composition according to any one of the preceding claims, further comprising an antioxidant, preferably in an amount of from 0.001 to 5 wt%.

11. The oral pharmaceutical composition according to claim 10, wherein the antioxidant is selected from the group consisting of butylated hydroxyltoluene, butylated hydroxyl anisole, alpha- tocopherol (Vitamin E), ascorbyl palmitate, ascorbic acid, sodium ascorbate, ethylenediamino tetraacetic acid, cysteine hydrochloride, citric acid, sodium citrate, sodium bisulfate, sodium metabisulfite, lecithin, propyl gallate, sodium sulfate, monothioglycerol and mixtures thereof.

12. The oral pharmaceutical composition according to claim 11, wherein the antioxidant is selected from the group consisting of alpha- tocopherol (Vitamin E), monothioglycerol, ascorbic acid, citric acid and mixtures thereof.

13. The oral pharmaceutical composition according to any one of the preceding claims, wherein the pharmaceutical

formulation is a solid at 20 °C and 1 atm.

**14.** The oral pharmaceutical composition according to any one of the preceding claims, wherein the oral pharmaceutical composition is an oral dosage form selected from the group consisting of mucoadhesive gel, a tablet, a powder, a liquid gel capsule, solid capsule, an oral solution, granule, or extrudates.

**15.** The oral pharmaceutical composition according to any one of the preceding claims, wherein the pharmaceutical formulation is only in the core and the modified-release agent is only in the shell.

**16.** The oral pharmaceutical composition according to any one of the preceding claims, wherein the oral pharmaceutical composition is an oral dosage form selected from the group consisting of a liquid gel capsule and a solid capsule.

## Patentansprüche

**1.** Orale pharmazeutische Zusammensetzung, umfassend:

einen Kern, der eine pharmazeutische Formulierung umfasst; und
eine Hülle, die zumindest ein Mittel mit modifizierter Freisetzung ausgewählt aus der Gruppe bestehend aus einem Copolymer aus Methacrylsäure und Methacrylat, einem Copolymer aus Methacrylsäure und Methylmethacrylat, einem Copolymer aus Methacrylsäure und Ethylacrylat, Hydroxypropylmethylcelluloseacetatsuccinat (HPMC-AS), Hydroxypropylmethylcellulosephthalat (HPMCP), Polyvinylacetatphthalat (PVAP), einem Copolymer aus Methylvinylether und Maleinsäureanhydrid, Celluloseacetatphthalat (CAP), Celluloseacetatbutyrat (CAB), Celluloseacetattrimellitat (CAT), Celluloseacetatsuccinat (CAS), Ethylcellulose, Methylcellulose, Schellack, Gellangummi, Zein, Alginsäure und Wachsen umfasst;
wobei die pharmazeutische Formulierung Folgendes umfasst:

Cannabidiol in einer Menge von 10 bis 50 Gew.-%;
ein Lösungsmittel in einer Menge von 20 bis 50 Gew.-%, wobei das Lösungsmittel ausgewählt ist aus der Gruppe bestehend aus Diacetin, Propylenglycol, Triacetin, Monoacetin, Propylenglycoldiacetat, Triethylcitrat und Mischungen davon; und
zumindest ein Poloxamer in einer Menge von 30 bis 60 Gew.-%, wobei das Poloxamer Poloxamer 124 oder Poloxamer 188 oder eine Mischung davon ist; und
wobei die Formulierung ölfrei ist.

**2.** Orale pharmazeutische Zusammensetzung nach Anspruch 1, wobei die pharmazeutische Formulierung zwei Poloxamere umfasst.

**3.** Orale pharmazeutische Zusammensetzung nach Anspruch 2, wobei die zwei Poloxamere Poloxamer 124 und Poloxamer 188 sind.

**4.** Orale pharmazeutische Zusammensetzung nach einem der vorhergehenden Ansprüche, wobei das Lösungsmittel ausgewählt ist aus der Gruppe bestehend aus Propylenglycol, Propylenglycoldiacetat, Triethylcitrat und Mischungen davon.

**5.** Orale pharmazeutische Zusammensetzung nach einem der vorhergehenden Ansprüche, wobei das Lösungsmittel ausgewählt ist aus der Gruppe bestehend aus Propylenglycol, Triethylcitrat und Mischungen davon.

**6.** Orale pharmazeutische Zusammensetzung nach einem der vorhergehenden Ansprüche, wobei das Lösungsmittel Triethylcitrat ist.

**7.** Orale pharmazeutische Zusammensetzung nach einem der vorhergehenden Ansprüche, wobei das Lösungsmittel in einer Menge von 20 bis 30 Gew.-% vorhanden ist.

**8.** Orale pharmazeutische Zusammensetzung nach einem der vorhergehenden Ansprüche, wobei das Cannabidiol synthetisch oder hochgereinigt aus seiner natürlichen Quelle ist.

**9.** Orale pharmazeutische Zusammensetzung nach einem der vorhergehenden Ansprüche, wobei das Cannabidiol in

einer Menge von 20 bis 30 Gew.-% vorhanden ist.

**10.** Orale pharmazeutische Zusammensetzung nach einem der vorhergehenden Ansprüche, ferner umfassend ein Antioxidans, bevorzugt in einer Menge von 0,001 bis 5 Gew.-%.

**11.** Orale pharmazeutische Zusammensetzung nach Anspruch 10, wobei das Antioxidans ausgewählt ist aus der Gruppe bestehend aus butyliertem Hydroxyltoluol, butyliertem Hydroxylanisol, alpha-Tocopherol (Vitamin E), Ascorbylpalmitat, Ascorbinsäure, Natriumascorbat, Ethylendiaminotetraessigsäure, Cysteinhydrochlorid, Zitronensäure, Natriumcitrat, Natriumbisulfat, Natriummetabisulfit, Lecithin, Propylgallat, Natriumsulfat, Monothioglycerin und Mischungen davon.

**12.** Orale pharmazeutische Zusammensetzung nach Anspruch 11, wobei das Antioxidans ausgewählt ist aus der Gruppe bestehend aus alpha-Tocopherol (Vitamin E), Monothioglycerin, Ascorbinsäure, Zitronensäure und Mischungen davon.

**13.** Orale pharmazeutische Zusammensetzung nach einem der vorhergehenden Ansprüche, wobei die pharmazeutische Formulierung bei 20 °C und 1 atm ein Feststoff ist.

**14.** Orale pharmazeutische Zusammensetzung nach einem der vorhergehenden Ansprüche, wobei die orale pharmazeutische Zusammensetzung eine orale Dosierungsform ausgewählt aus der Gruppe bestehend aus mucoadhäsivem Gel, einer Tablette, einem Pulver, einer flüssigen Gelkapsel, festen Kapsel, einer oralen Lösung, Granulat oder Extrudaten ist.

**15.** Orale pharmazeutische Zusammensetzung nach einem der vorhergehenden Ansprüche, wobei die pharmazeutische Formulierung nur in dem Kern ist und das Mittel mit modifizierter Freisetzung nur in der Hülle ist.

**16.** Orale pharmazeutische Zusammensetzung nach einem der vorhergehenden Ansprüche, wobei die orale pharmazeutische Zusammensetzung eine orale Dosierungsform ausgewählt aus der Gruppe bestehend aus einer flüssigen Gelkapsel und einer festen Kapsel ist.

**Revendications**

**1.** Composition pharmaceutique orale comprenant :

un noyau qui comprend une formulation pharmaceutique ; et
une enveloppe qui comprend au moins un agent à libération modifiée choisi dans le groupe constitué d'un copolymère d'acide méthacrylique et de méthacrylate, d'un copolymère d'acide méthacrylique et de méthacrylate de méthyle, d'un copolymère d'acide méthacrylique et d'acrylate d'éthyle, de l'acétate succinate d'hydroxypropylméthylcellulose (HPMC-AS), du phtalate d'hydroxypropylméthylcellulose (HPMCP), de l'acétate phtalate de polyvinyle (PVAP), d'un copolymère d'éther méthylvinylique et d'anhydride maléique, de l'acétate phtalate de cellulose (CAP), de l'acétate butyrate de cellulose (CAB), de l'acétate trimellitate de cellulose (CAT), de l'acétate succinate de cellulose (CAS), de l'éthylcellulose, de la méthylcellulose, de la gomme shellac, de la gomme gellane, de la zéine, de l'acide alginique et des cires ;
dans laquelle la formulation pharmaceutique comprend :

un cannabidiol en une quantité de 10 à 50 % en poids ;
un solvant, en une quantité de 20 à 50 % en poids, dans laquelle le solvant est choisi dans le groupe constitué de la diacétine, du propylène glycol, de la triacétine, de la monoacétine, du diacétate de propylène glycol, du citrate de triéthyle et de mélanges de ceux-ci ; et
au moins un poloxamère en une quantité de 30 à 60 % en poids, dans laquelle le poloxamère est le poloxamère 124 ou le poloxamère 188 ou un mélange de ceux-ci ; et
dans laquelle la formulation est exempte d'huile.

**2.** Composition pharmaceutique orale selon la revendication 1, dans laquelle la formulation pharmaceutique comprend deux poloxamères.

**3.** Composition pharmaceutique orale selon la revendication 2, dans laquelle les deux poloxamères sont le poloxamère

124 et le poloxamère 188.

4. Composition pharmaceutique orale selon l'une quelconque des revendications précédentes, dans laquelle le solvant est choisi dans le groupe constitué du propylène glycol, du diacétate de propylène glycol, du citrate de triéthyle et de mélanges de ceux-ci.

5. Composition pharmaceutique orale selon l'une quelconque des revendications précédentes, dans laquelle le solvant est choisi dans le groupe constitué du propylène glycol, du citrate de triéthyle et de mélanges de ceux-ci.

6. Composition pharmaceutique orale selon l'une quelconque des revendications précédentes, dans laquelle le solvant est le citrate de triéthyle.

7. Composition pharmaceutique orale selon l'une quelconque des revendications précédentes, dans laquelle le solvant est présent en une quantité de 20 à 30 % en poids.

8. Composition pharmaceutique orale selon l'une quelconque des revendications précédentes, dans laquelle le cannabidiol est synthétique ou hautement purifié à partir de sa source naturelle.

9. Composition pharmaceutique orale selon l'une quelconque des revendications précédentes, dans laquelle le cannabidiol est présent en une quantité de 20 à 30 % en poids.

10. Composition pharmaceutique orale selon l'une quelconque des revendications précédentes, comprenant en outre un antioxydant, de préférence en une quantité de 0,001 à 5 % en poids.

11. Composition pharmaceutique orale selon la revendication 10, dans laquelle l'antioxydant est choisi dans le groupe constitué de l'hydroxyltoluène butylé, de l'hydroxylanisole butylé, de l'alpha-tocophérol (vitamine E), du palmitate d'ascorbyle, de l'acide ascorbique, de l'ascorbate de sodium, de l'acide éthylènediaminotétraacétique, du chlorhydrate de cystéine, de l'acide citrique, du citrate de sodium, du bisulfate de sodium, du métabisulfite de sodium, de la lécithine, du gallate de propyle, du sulfate de sodium, du monothioglycérol et de mélanges de ceux-ci.

12. Composition pharmaceutique orale selon la revendication 11, dans laquelle l'antioxydant est choisi dans le groupe constitué de l'alpha-tocophérol (vitamine E), du monothioglycérol, de l'acide ascorbique, de l'acide citrique et de mélanges de ceux-ci.

13. Composition pharmaceutique orale selon l'une quelconque des revendications précédentes, dans laquelle la formulation pharmaceutique est un solide à 20 °C et 1 atm.

14. Composition pharmaceutique orale selon l'une quelconque des revendications précédentes, dans laquelle la composition pharmaceutique orale est une forme posologique orale choisie dans le groupe constitué d'un gel mucoadhésif, d'un comprimé, d'une poudre, d'une capsule de gel liquide, d'une capsule solide, d'une solution orale, d'un granulé ou d'extrudés.

15. Composition pharmaceutique orale selon l'une quelconque des revendications précédentes, dans laquelle la formulation pharmaceutique se trouve uniquement dans le noyau et l'agent à libération modifiée se trouve uniquement dans l'enveloppe.

16. Composition pharmaceutique orale selon l'une quelconque des revendications précédentes, dans laquelle la composition pharmaceutique orale est une forme posologique orale choisie dans le groupe constitué d'une capsule de gel liquide et d'une capsule solide.

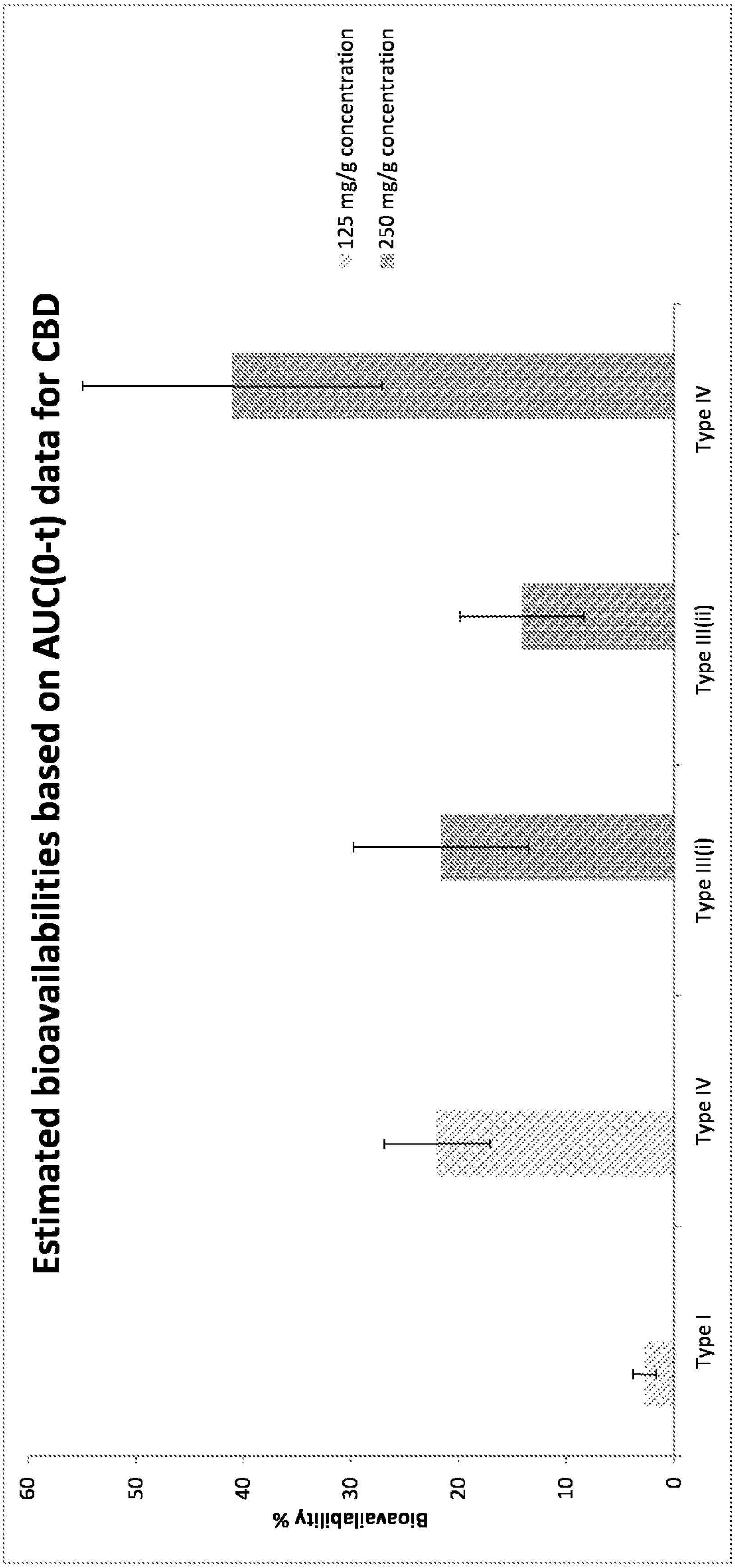
Estimated bioavailabilities based on AUC(0-t) data for CBD
125 mg/g concentration
250 mg/g concentration
Bioavailability %
60
50
40
30
20
10
0
Type I
Type IV
Type III(i)
Type III(ii)
Type IV

Figure 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012033478 A
- WO 2015184127 A
- GB 2017051943 W
- CN 103110582
- CN 101040855
- US 2012183606 A
- WO 2017072774 A
- AU 2016203127
- US 2016184258 A
- US 2015111939 A


### Non-patent literature cited in the description


- **S. ZHORNITSKY** ; **S. POTVIN**. *Pharmaceuticals*, 2012, vol. 5, 529-552
- **A. J. POORTMAN** ; **H. HUIZER**. *Forensic Science International*, 1999, vol. 101, 1-8
- **THUMMA S et al.** *European Journal of Pharmaceutics and Biopharmaceutics*, 01 October 2008, vol. 70 (2), 605-614
- **EDWARD MAA et al.** *Epilepsia*, 01 June 2014, vol. 55 (6), 783-786
- **C.W. POUTON**. *Eur. J. Pharm. Sci.*, 2000, vol. 11 (2), S93-S98
- *FABAD J. Pharm. Sci.*, 2013, 55-64
- Guidance for Industry Botanical Drug Products Draft Guidance. US Department of Health and Human Services, Food and Drug Administration Centre for Drug Evaluation and Research, August 2000